# EUROPEAN PATENT APPLICATION

(11) **EP 1 443 112 A2**
(43) Date of publication of application: **04.08.2004**
(21) Application number: 04076092.8
(22) Date of filing: 05.06.1996
(51) Int. Cl.: C12N 15/19, C07K 14/52

(54) **Macrophage activating factors derived from cloned vitamin D binding protein**

(30) Priority: 07.06.1995 US 478121; 19.03.1996 US 618485
(62) Divisional of application: 96921279.4
(71) Applicant: Yamamoto, Nobuto, Philadelphia, PA 19126 (US)
(72) Inventor: Yamamoto, Nobuto, Philadelphia, PA 19126 (US)
(74) Representative: Shaw, Laurence

(57) **Abstract**

A process for producing a cloned macrophage activating factor (GcMAFc) comprises the steps of:
a) cloning a Gc1 isoform into a baculovirus vector;
b) expressing the cloned Gc1 isoform thereby producing the cloned protein (Gc1), wherein the Gc1 protein comprises approximately 458 amino acids with a molecular weight of approximately 52000 Da and three distinct domains,
c) contacting cloned Gc protein *in vitro* with immobilized β-galactosidase and sialidase; and
d) obtaining the cloned macrophage activating factor (GcMAFc).

## Description

### FIELD OF THE INVENTION

This invention relates to potent macrophage activating factors, prepared by oligosaccharide digestion of the cloned vitamin D binding protein (Gc protein) and the cloned Gc protein domain III, and the use of these macrophage activating factors for various cancers, HIV-infection and osteoporosis, and as a adjuvant for immunization and vaccination and also development of diagnostic and prognostic methods utilizing the specific enzyme, α-N-acetylgalactosaminidase detected in the blood stream of cancer and HIV-infected patients.

| **TABLE OF TERMS** | |
|---|---|
| Gc protein | Vitamin D₃-binding protein |
| MAF | macrophage activating factor |
| GcMAF | Gc protein-derived macrophage activating protein |
| GcMAFc | cloned Gc protein-derived macrophage activating factor |
| Gc domain III | domain III region of Gc protein |
| CdMAF | cloned domain III-derived macrophage activating factor |
| NagAg | α-N-acctylgalactosaminidase as an antigen |

### SUMMARY OF THE INVENTION

Vitamin D-binding protein (Gc protein) and its small domain (approximately 18% length of the Gc peptide also known as domain III) were cloned via a baculovirus vector. The cloned Gc protein and the cloned domain (Cd) peptide were treated with immobilized β-galactosidase and sialidase to yield macrophage. activating factors, GcMAFc and CdMAF, respectively. These cloned macrophage activating factors and GcMAF are to be used for cancer therapy, HIV-infection and osteoporosis, and may also be used as a adjuvant for immunization and vaccination. Methods and an antibody-sandwich ELISA kit were developed to detect serum or plasma α-N-acetylgal-actosaminidase in cancer and HIV-infected patients and used as diagnostic and prognostic indices.

### DESCRIPTION OF THE DRAWINGS

Other objects and many attendant features of this invention will become readily appreciated as the same becomes better understood by reference to the following detailed description when considered in connection with the accompanying drawings wherein:
Fig. 1a is a schematic illustration of the formation of macrophage activating factor (MAF).
Fig. 1b is a schematic illustration of the deglycosylation of Gc protein in a cancer or HIV-infected patient's blood stream.
Fig. 2 shows the amino acid sequence of cloned GcMAF which is SEQ ID NO:1 which is the entire cloned Gc protein.
Fig. 3 shows the construction of the DNA fragment encoding the leader sequence of EcoRI fragment E1 and domain III regions of the Gc protein; A, the entire cDNA for Gc protein; B, the construct to be inserted into the non-fusion vector; the shaded area indicates the compressed regions of about 1,000 base pairs (bp).
Fig. 4 shows the 89 amino acid sequence, SEQ ID NO:2, of the cloned domain III (CdMAF₁), using the non-fusion vector.
Fig. 5 shows the baculovirus fusion vector for cloning the domain III of Gc protein.
Fig. 6 shows the 94 amino acid sequence, SEQ ID NO:3, of the cloned domain III (CdMAF₂), using the fusion vector.
Fig. 7 shows the correlation between plasma α-N-acerylgalactosaminidase activity and tumor burden (total cell counts) in the peritoneal cavity of Ehrlich ascites tumor.
Fig. 8A shows the therapeutic effect of GcMAF in accordance with the present invention on adult persons suffering from prostate cancer.
Fig. 8B shows the therapeutic effect of GcMAF in accordance with the present invention on adult persons suffering from breast cancer.
Fig. 8C shows the therapeutic effect of GcMAF in accordance with the present invention on adult persons suffering from colon cancer.

### BACKGROUND OF THE INVENTION

### A. Inflammatory Response Results In Activation of Macrophages

Inflammation results in the activation of macrophages. Inflamed lesions release lysophospholipids. The administration into mice of small doses (5-20 µg/mouse) of lysophosphatidylcholine (lyso-Pc) and other lysophospholipids induced a greatly enhanced phagocytic and superoxide generating capacity of macrophages (Ngwenya and Yamamoto, Proc. Soc. Exp. Biol. Med. 193:118, 1990; Yamamoto et al., Inf. Imm. 61:5388, 1993; Yamamoto et al., Inflammation. 18:311, 1994).

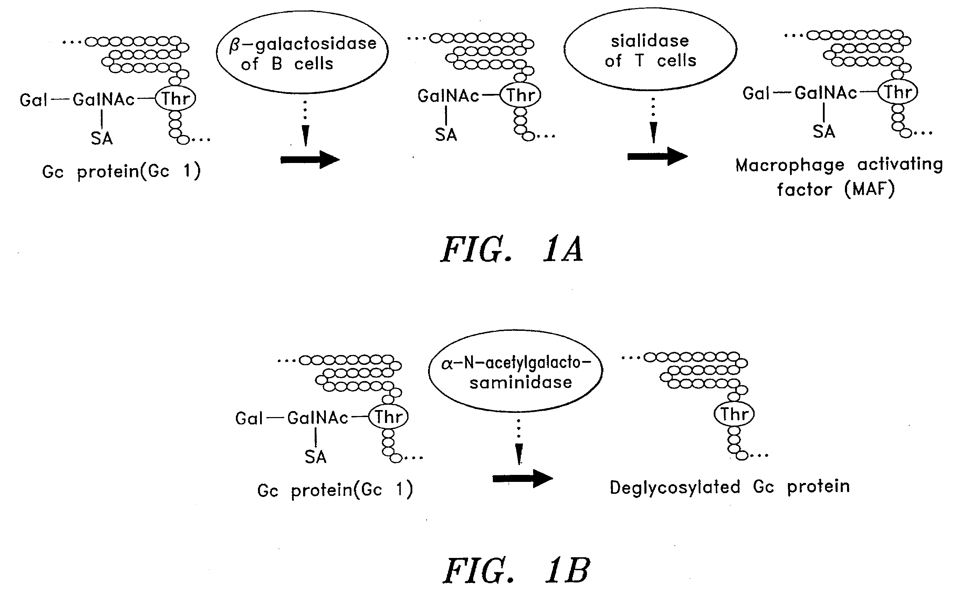

This macrophage activation requires participation of B and T lymphocytes and serum vitamin D binding protein (DBP; human DBP is known as Gc protein). In vitro activation of mouse peritoneal macrophages by lyso-Pc requires the step-wise modification of Gc protein by β-galactosidase of lyso-Pc-treated B cells and sialidase of T cells to generate the macrophage activating factor (MAF), a protein with N-acetylgalactosamine as the remaining sugar moiety (Fig 1a) (Yamamoto et al., Proc. Natl. Acad. Sci. USA. 88:8539, 1991; Yamamoto et al., J. Immunol. 151:2794, 1993; Naraparaju and Yamamoto, Immunol. Lett. 43:143, 1994). Thus, Gc protein is a precursor for MAF.

Incubation of Gc protein with immobilized β-galactosidase and sialidase generates a remarkably high titered MAF (GcMAF) (Yamamoto et al., Proc. Natl. Acad. Sci. USA. 88:8539, 1991; Yamamoto et al., J. Immunol. 151:2794, 1993; Naraparaju and Yamamoto, Immunol. lett. 43:143, 1994; US Patent # 5,177,002; US Patent # 5,326,749). Administration of a minute amount (10 pg/mouse; 100 ng/human) of GcMAF resulted in greatly enhanced phagocytic and superoxide generating capacities of macrophages.

When peripheral blood monocytes/macrophages (designated as macrophages hereafter) of 326 patients bearing various types of cancer were treated in vitro with 100 pg GcMAF/ml, the macrophages of all cancer patients were activated for phagocytic and superoxide generating capacity. This observation indicates that cancer patient macrophages are capable of being activated. However, the MAF precursor activity of plasma Gc protein was lost or reduced in approximately 70% of this cancer patient population. Lost or reduced precursor activity of Gc protein was found to be due to deglycosylation of plasma Gc protein by α-N-acetylgalactosaminidase detected in the cancer patient's blood stream. Deglycosylated Gc protein cannot be converted to MAF (Fig. 1b). Thus, the loss of the MAF precursor activity of Gc protein prevents generation of MAF. Therefore, macrophage activation cannot develop in certain cancer patients. Since macrophage activation is the first step in the immune development cascade, such cancer patients become immunosuppressed. This may explain at least in part why cancer patients die from overwhelming infection.

Similarly, when peripheral blood macrophages of 196 HIV-infected/AIDS patients were treated in vitro with 100 pg GcMAF/ml, the macrophages of all patients were activated for phagocytic and superoxide generating capacity. However, the MAF precursor activity of plasma Gc protein was low in approximately 35% of the HIV-infected patient population. As in cancer patients, these patients' plasma Gc protein is deglycosylated by α-N-acetylgalactosaminidase detected in HIV-infected patients. This mechanism explains why advanced HIV-infected (AIDS) patients are severely immunosuppressed.

Both cancer and HIV-infected patients having severely decreased precursor activity of plasma Gc protein carried large amounts of α-N-acetylgalactosaminidase while patients having moderately decreased precursor activity had moderate levels of plasma α-N-acetylgalactosaminidase activities. Patients with high precursor activity, including asymptomatic HIV-infected patients, had low but significant levels of plasma α-N-acetylgalactosaminidase activity. Since a large amount (260 µg/ml) of Gc protein exists in the blood stream, a low level of the enzyme does not affect the precursor activity. Nevertheless, α-N-acetylgalactosaminidase activity was found in plasmas of all cancer and HIV-infected patients and had an inverse correlation with the precursor activity of their plasma Gc protein. Thus, increase in patient serum or plasma α-N-acetylgalactosaminidase activity is responsible for decrease in the precursor activity of plasma Gc protein. These observations led me to propose that serum or plasma α-N-acetylgalactosaminidase plays a role in immunosuppression in cancer and HIV-infected/AIDS patients. Thus, patient serum or plasma α-N-acetylgalactosaminidase activity can serve as a diagnostic and prognostic index.

### B. A Defect in Macrophage Activation Cascade Manifests Osteoporosis

The inflammation-primed macrophage activation process appears to be the major macrophage activation cascade, which is shared by other phagocytes such as osteoclast and monocytes. A defect in the inducible β-galactosidase of B lymphocytes in the macrophage activation cascade causes dysfunctional osteoclasts leading to manifestation of osteoporosis (Yamamoto et al., J. Immunol.152·5100, 1994), lacking or delaying bone marrow formation in newborn.

Autosomal recessive osteoporosis is characterized by an excess accumulation of bone throughout the skeleton as a result of dysfunctional osteoclasts, resulting in reduced bone resorption (Marks, Clin. Orthop. 189:239, 1984). In animal models of osteoporosis, depending on the degree of osteoclast dysfunction, marrow cavity development and tooth eruption are either delayed or more commonly absent (Marks, Am. J. Med. Genet. 34:43, 1989). In human infantile osteoporosis, death occurs within the first decade of life usually overwhelming infection (Reeves, Pediatrics. 64:202,1979), indicating immunosuppression. Accumulated evidence suggests that deficient or dysfunctional osteoclasts in osteopetrotic mammals including humans are often accompanied by deficiencies or dysfunctions of macrophages. The studies of the present inventor on the activation of both osteoclasts and macrophages in the osteopetrotic mutations revealed that osteoclasts and macrophages can be activated by a common signaling factor, the macrophage activating factor and that a defect in β-galactosidase of B cells incapacitates the generation process of macrophage activating factor (Yamamoto et al., J. Immunol. 152:5100, 1994). Since GcMAF and its cloned derivatives bypass the functions of lymphocytes and Gc protein and act directly on macrophages and osteoclasts, administration of these factors into osteopetrotic hosts should rectify the bone disorder. In fact the present inventor has recently found that four administrations of GcMAF or cloned macrophage activating factor (GcMAFc) (100 pg/week) to the op mutant mice beginning at birth for four weeks resulted in the activation of both macrophages and osteoclasts and subsequent resorption of the excess skeletal matrix and increased size of marrow cavity.

### C. Therapeutic Application of GcMAF and its Cloned Derivatives on Cancer

Despite defects in the macrophage activation cascade in cancer, HIV-infected and osteopetrotic patients, GcMAF bypasses the functions of lymphocytes and Gc protein and acts directly on macrophages (or osteoclasts) for activation. Macrophages have a potential to eliminate cancerous cells and HIV-infected cells when activated. When cancer patients were treated with 100 ng GcMAF/patient weekly for several months, GcMAF showed remarkable curative effects on a variety of human cancer indiscriminately.

Instead of obtaining of GcMAF from human blood source, it can be obtained from the cloned Gc protein or its small domain responsible for macrophage activation. The cloned Gc protein requires an eukaryotic vector/host capable of the glycosylation of the cloned products. The Gc protein with a molecular weight of approximately 52,000 and 458 amino acid residues) is a multi-functional protein and carries three distinct domains (Cooke and Haddad, Endocrine Rev., 10:294, 1989).

Domain I interacts with vitamin D while domain III interacts with actin (Haddad et al., Biochem., 31:7174, 1992). Chemically and proteolytically fragmented Gc protein enabled me to indicate that the smallest domain, domain III, contains 80 amino acid residues including the site for glycosylation, an essential peptide for macrophage activation. Accordingly, I cloned both Gc protein and the entire domain III peptide, by the use of a baculovirus vector and an insect host, and treated them with the immobilized β-galactosidase and sialidase to yield potent macrophage activating factors, designated GcMAFc and CdMAF, respectively. Like GcMAF, these cloned GcMAFc and CdMAF appear to have remarkably curative effects on a variety of cancers.

### D. A potent adjuvant activity of GcMAF for immunization with antigens or vaccines

Macrophages are antigen presenting cells. Macrophages activated by GcMAF rapidly phagocytize target antigens or cells and presented the processed antigens to antibody producing cells. I observed a rapid development of a large amount of antibody secreting cells immediately (1 to 4 days) after inoculation of small amount of GcMAF (100 pg/mouse) and sheep erythrocytes (SRBC). This finding indicates that GcMAF and its cloned derivatives, GcMAFc and CdMAF, should serve as a potent adjuvant for immunization and vaccination.

### DESCRIPTION OF THE METHODS FOR GENE CLONING FOR MACROPHAGE ACTIVATING FACTORS

### A. Cloning of the cDNA of Gc Protein into an Insect Virus.

A full length cDNA encoding the human Gc protein was isolated from a human liver cDNA library in bacteriophage λgt11 (Clontech, Palo Alto, CA) by the use of a pico Blue_{TM} immunoscreening kit available from Stratagene of La Jolla, CA. The baculoviral expression system in the insect cells takes advantages of several facts about the polyhedron protein: (a) it is expressed to very high levels in infected cells where it constitutes more than half of the total cellular protein late in the infection cycle; (b) it is nonessential for infection or replication of the virus, meaning that the recombinant virus does not require any helper function; (c) viruses lacking the polyhedron gene have distinct plaque morphology from viruses containing the cloned gene; and d) unlike bacterial cells, the insect cell efficiently glycosylates the cloned gene products.

One of the beauties of this expression system is a visual screen allowing recombinant viruses to be distinguished and quantified. The polyhedron protein is produced at very high levels in the nuclei of infected cells late in the viral infection cycle. Accumulated polyhedron protein forms occlusion bodies that also contain embedded virus particles. These occlusion bodies, up to 15 µm in size, are highly refractile, giving them a bright shiny appearance that is readily visualized under a light microscope. Cells infected with recombinant viruses lack occlusion bodies. To distinguish the recombinant virus from a wild-type virus, the transfection supernatant (recombinant containing virus lysate) is plaqued onto a monolayer of insect cells. Plaques are then screened under a light microscope for the presence (indicative of wild-type virus) or absence (indicative of recombinant virus) of occlusion bodies.

In addition, the baculoviral expression system uses a helper-independent virus that can be propagated to high titers in insect cells adapted for growth in suspension cultures, making it possible to obtain large amounts of recombinant protein with relative ease. The majority of the overproduced protein remains soluble in insect cells by contrast with the insoluble proteins often obtained from bacteria. Furthermore, the viral genome is large (130 kbp) and thus can accommodate large segments of foreign DNA.

### 1) Choice of baculoviral vector.

All available baculoviral vectors arc pUC-based and confer ampicillin resistance. Each contains the polyhedron gene promoter, variable lengths of polyhedron coding sequence, and insertion site(s) for cloning the foreign gene of interest flanked by viral sequences that lie 5' to the promoter and 3' to the foreign gene insert. These flanking sequences facilitate homologous recombination between the vector and wild-type baculoviral DNA (Ausubel et al., Current Protocols in Mol. Biol. 1990). The major consideration when choosing the appropriate baculoviral expression vector is whether to express the recombinant as a fusion or non-fusion protein. Since glycosylation of the Gc peptide requires a leader signal sequence for transfer of the peptide into the endoplasmic reticulum, the cDNA containing initiation codon (-16 Met) through the leader sequence to the +1 amino acid (leu) of the native Gc protein should be introduced to the non-fusion vector with a polylinker carrying the EcoRI site, pLV1393 (Invitrogen, San Diego, CA).

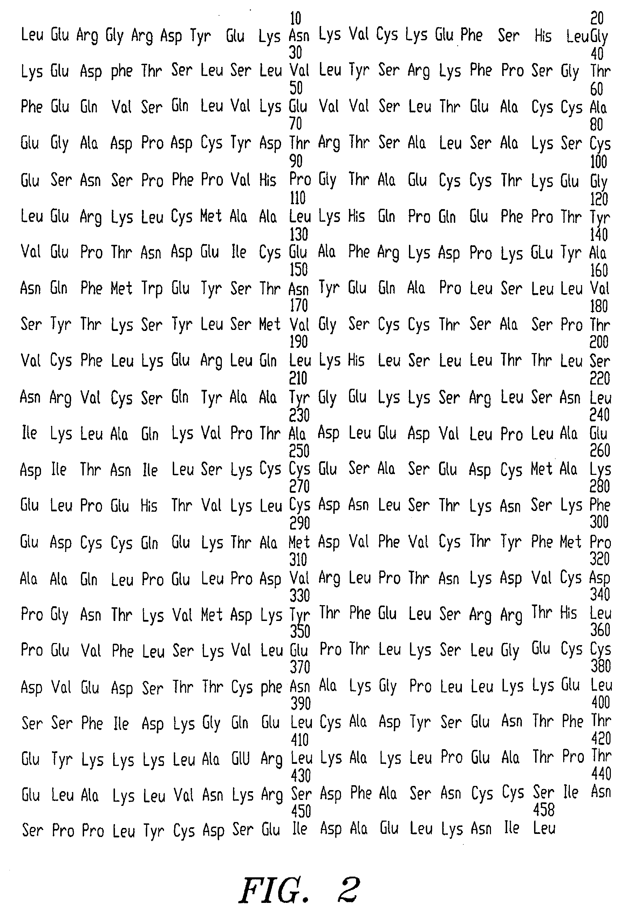

During partial digestion of the cDNA for Gc protein in λgt11 with EcoRI enzyme, a full length Gc cDNA with EcoRI termini was isolated electrophoretically, mixed with EcoRI-cut pVL1393, and ligated with T4 ligase. This construct in the correct orientation should express the entire Gc peptide, a total of 458 amino acids (Fig. 2). To obtain the correct construction, competent E. coli HB101 cells were transformed with pVL vector and selected for transformants on Luria broth agar plates containing ampicillin (LB/ampicillin plates). The DNA was prepared for the sequencing procedure to determine which colony contains the insert or gene with the proper reading orientation, by first searching for the 3' poly A stretch. The clones with 3' ply A (from the poly A tail of mRNA) were then sequenced from the 5' end to confirm the correct orientation of the full length DNA for the Gc peptide.

### 2) Co-transfection of insect cells with the cloned plasmid DNA and wild-type viral DNA

A monolayer (2.5x10⁶ cells in each of 25-cm² flasks) of Spodoptera frugiperda (Sf9) cells was co-transfected with a cloned plasmid (vector) DNA (2 µg) and a wild-type (AcMNPV) baculoviral DNA (10 µg) in 950 µl transfection buffer (Ausubel et al., In Curr Protocols in Mol. Biol. 1990). When the cells were cultured for 4 or 5 days, the transfection supernatant contained recombinant viruses.

### 3) Identification of recombinant baculovirus

The co-transfection lysates were diluted 10⁴, 10⁵ or 10⁶ and plated on Sf9 cells for cultivation for 4 to 6 days. After the plaques were well formed, plaques containing occlusion-negative cells were identified at a frequency of 1.3%. Several putative recombinant viral plaques were isolated and twice re-plaqued for purification. Pure recombinant viral plaque clones were isolated.

### B. Analysis of Protein of Interest from Recombinant Baculovirus

### 1) Preparation of recombinant virus lysate

An insect cell Sf9 monolayer (2.5x10⁶ cells per 25-cm² flask) was infected with a recombinant virus clone and cultured in 5 ml GIBCO scrum-free medium (from GIBCO Biochemicals, Rockville, MD) or medium supplemented with 0.1% egg albumin to avoid contamination of serum bovine vitamin D binding protein. The culture flasks were incubated at 27°C and monitored daily for signs of infection. After 4 to 5 days, the cells were harvested by gently dislodging them from the flask and the cells and culture medium were transferred to centrifuge tubes and centrifuged for 10 min at 1000 x g, 4°C. To maximize infection for recombinant protein production, Sf9 cells were grown in a 100-ml spinner suspension culture flask with 50 ml complete medium up to about 2 x 10⁶ cells/ml. The cells were harvested, centrifuged at 1000 x g for 10 min and resuspended in 10 to 20 ml serum-free medium containing recombinant virus at a multiplicity of infection (MOI) of 10. After 1 hour of incubation at room temperature, the infected cells were transferred to a 200-ml spinner flask containing 100 ml serum-free medium and incubated for 40 hr. More than 40% of the protein secreted was the protein of interest. The protein in the supernatant was isolated.

### 2) Qualitative estimation of the protein of interest

Coomassie Blue staining of the SDS-polyacrylamide gel, loading 20 to 40 µg total cell protein per lane, was used to estimate the quantity of expressed protein. Because the samples contain cellular proteins, the recombinant protein was readily detected by comparison with uninfected cellular proteins.

### 3) Enzymatic conversion of the cloned Gc protein to macrophage activating factor (GcMAFc).

The cloned Gc protein (2 µg) with a molecular weight of 52,000 and 458 amino acid residues (Fig. 2) was isolated by a vitamin D-affinity column (Link et al. Anal. Biochem. 157:262, 1986) and treated with immobilized β-galactosidase and sialidase. The resultant cloned macrophage activating factor (GcMAFc) was added to mouse and human macrophages and assayed for phagocytic and superoxide generating capacity. Incubation of macrophages with 10 pg GcMAFc/ml for 3 hours resulted in a 5-fold increased phagocytic activity and a 15-fold increase in the superoxide generating capacity of macrophages.

### C. Subcloning of a Domain-Required for Macrophage Activation

### I. Cloning procedure I: Non-fusion vector.

### 1) Cloning the domain responsible for macrophage activation (CdMAF)

The entire cDNA sequence for Gc protein in λgt11, including 76 bp of the upstream 5' flanking region and 204 bp of the 3' flanking stretch, was fragmented by EcoRI to yield four restriction fragments designated E1, 120; E2, 314; E3, 482; and E4, 748 bp, respectively. Each was cloned into the EcoRI site of the plasmid pSP65 from Promega (Madison, WI) by the method of Cooke and David (J. Clin. Invest., 76 2420, 1985). Although I found that a region less than one half of the domain III was found to be responsible for macrophage activation, small segments less than 40 amino acid residues cannot be expressed in the insect cells. Moreover, short peptides are rapidly degraded by proteases in human plasma, and thus are not clinically useful. Accordingly, the entire domain III (approximately 80 amino acid residues) should be subcloned into an insect virus where I anticipate the efficient production and glycosylation of the peptide in the infected cells.

### 2) Subcloning cDNA fragment into the polyhedron gene of baculovirus

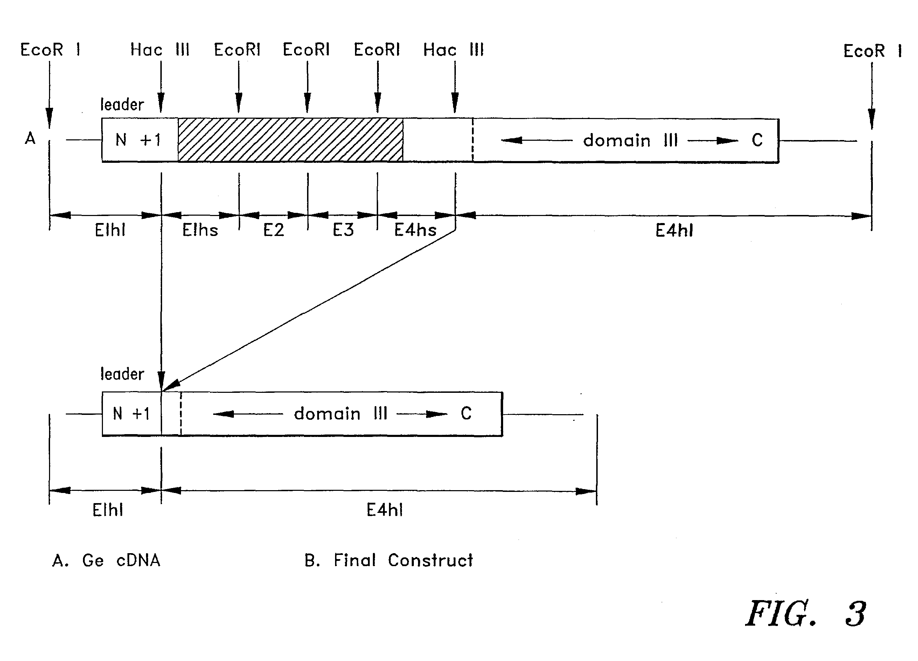

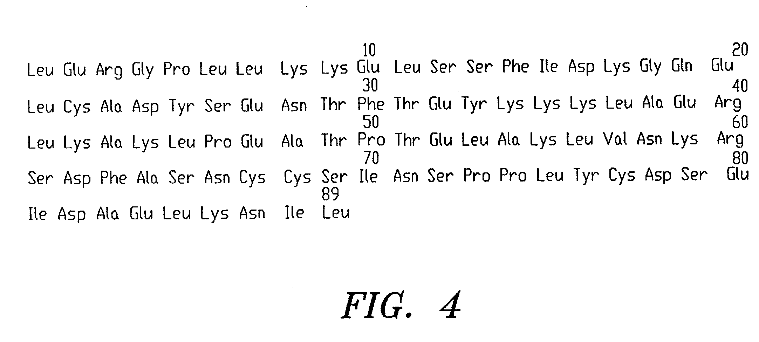

Since the glycosylation of a peptide requires a leader signal sequence for transfer of the peptide into the endoplasmic reticulum, the DNA segment of E1 containing the initiation codon (-16 Met) through the leader sequence to the +1 amino acid (Leu) of the native Gc protein should be introduced into the vector. Because this segment carries the initiation codon for the Gc protein, non-fusion vector, pVL1393 (Invitrogen, San Diego, CA) was used. A segment containing the initiation codon-leader sequence of the cDNA clone E1 and a cDNA segment coding for 85 C-terminal amino acids (the entire domain III) plus 3' non-coding stretch of the cDNA clone E4 were ligated together and cloned into the EcoRI site of the insect virus pVL vector. To achieve this construct, both E1 and E4 DNA were fragmented with HaeIII to yield two fragments each; E1hl (87 bp), E1hs (33 bp) and E4hs (298 bp), E4hl (450 bp), respectively. Both the larger fragments E1hl and E4h1 were isolated electrophoretically, mixed with EcoRI-cut pVL, and ligated with T4 ligase, as shown in Fig. 3. This construct in the correct orientation should express the entire domain III, a total of 89 amino acids, including the 4 amino acids of E1hl, also referred to herein as CDMAF₁ as shown in Fig. 4. To obtain the correct construction, competent E. coli HB101 cells are transformed with the pVL vector and selected for transformants on LB/ampicillin plates. DNA was prepared for sequencing procedures to determine which colony contains the construct with proper reading orientation by first searching for the 3' poly dA stretch. Those clones with 3' poly dA (from the poly A tail of mRNA) were then sequenced from the 5' end to confirm the correct orientation of the E1hl fragment. I found that the vector contains the entire construct (domain III) in the correct orientation.

### 3) Isolation of recombinant baculovirus, purification of the cloned domain peptide (Cd) and enzymatic generation of the cloned macrophage activating factor (CdMAF)

Monolayers (2.5x10⁶ cells in each of 25-cm² flasks) of Spodoptera frugiperda (Sf9) cells were co-transfected with cloned plasmid DNA (2 µg) and wild-type (AcMNPV) baculoviral DNA (10 µg) in 950 µl transfection buffer. Recombinant baculovirus plaques were isolated and used for production of the Gc domain III peptide in insect cells. This cloned domain with a molecular weight (MW) of 10,000 and 89 amino acids as shown in Fig. 4, was purified by actin-affinity column (Haddad et al., Biochemistry 31:7174, 1992). Two µg of the cloned domain (Cd₁) peptide was treated with immobilized β-galactosidase and sialidase to yield a cloned macrophage activating factor, designated as CdMAF₁.

### II. Cloning procedure II: Fusion vector.

### 1) Cloning the domain responsible for macrophage activation (CdMAF)

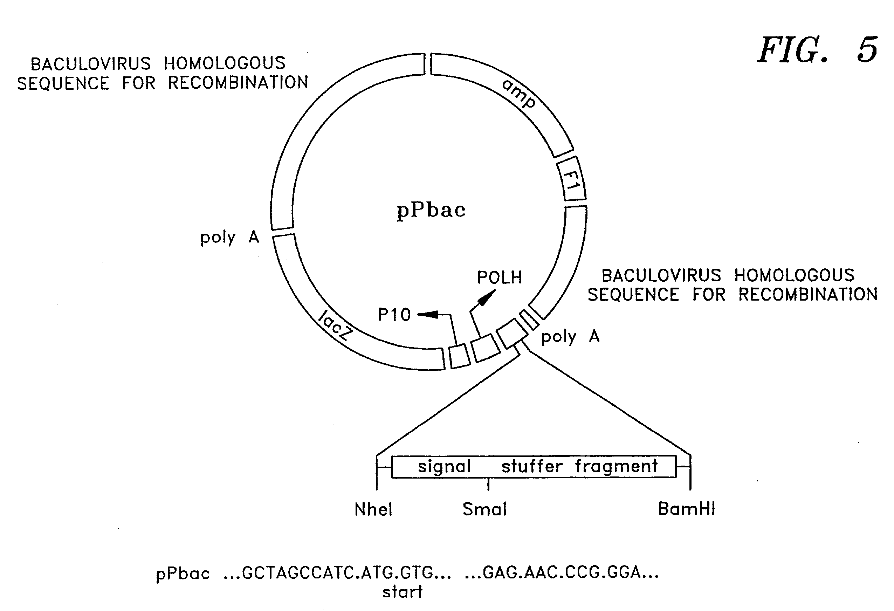

A baculovirus fusion vector, pPbac vector (Stratagene, La Jolla, CA), contains human placental alkaline phosphatase secretory signal sequences that direct the nascent cloned peptide toward the secretory pathway of the cells leading to secretion into culture media. The signal sequence is cleaved off by signal-sequence peptidase as the nascent cloned peptide is channeled toward the secretory pathway of the host insect cells leading to secretion of the cloned domain (Cd) peptide. Fig. 5 depicts that the vector carries the stuffer fragment for gene substitution and lacZ gene for identification of the gene insertion.

The stuffer fragment of pPbac vector was excised by digesting the vector DNA with restriction enzymes Smal and BamHI and was removed by electroelution. The E4 cDNA fragment of the Gc protein was digested with HaeIII and BamHI, yielding a fragment practically the same as E4h1 (see Fig. 3). This fragment was mixed with the above pPbac vector and ligated with T4 ligase. This strategy not only fixes the orientation of ligation but also fuses the fragment with the reading frame. The E. coli DH5aF' cells were transformed with the reaction mixture. The cloned DNA insert was isolated from a number of colonies after digestion with HaeIII and BamHI. The insert was confirmed by sequencing. The sequence confirmed the correct orientation.

### 2) Isolation of recombinant baculovirus by transfection of Sf9 insect cells with wild type baculovirus and the cloned DNA insert.

For the transfection of insect cells (Spodoptera frugiperda ,Sf9), linear wild type (AcMNPV) baculoviral DNA and inscctin liposomes (Invitrogen, San Diego, CA) have been used. Liposome-mediated transfection of insect cells is the most efficient transfection method available. For transfection to a monolayer of Sf9 cells (2 X 10⁶) in a 60 mm dish, a mixture of the following was gently added:
3 µg cloned plasmid DNA
10 µl linear wild type baculovirus (AcMNPV) DNA (0.1 µg/µl)
1 ml medium
29 µl insectin liposomes

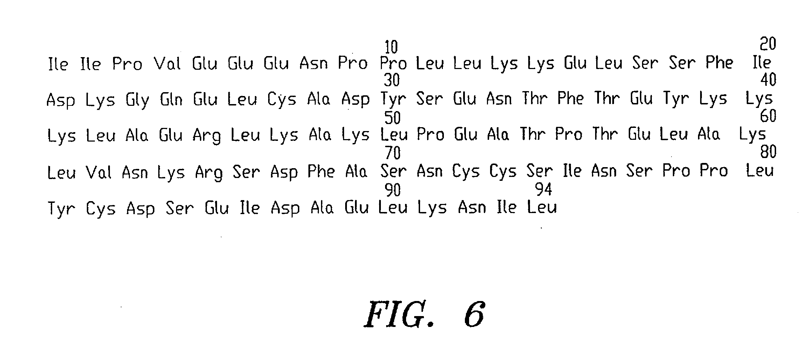

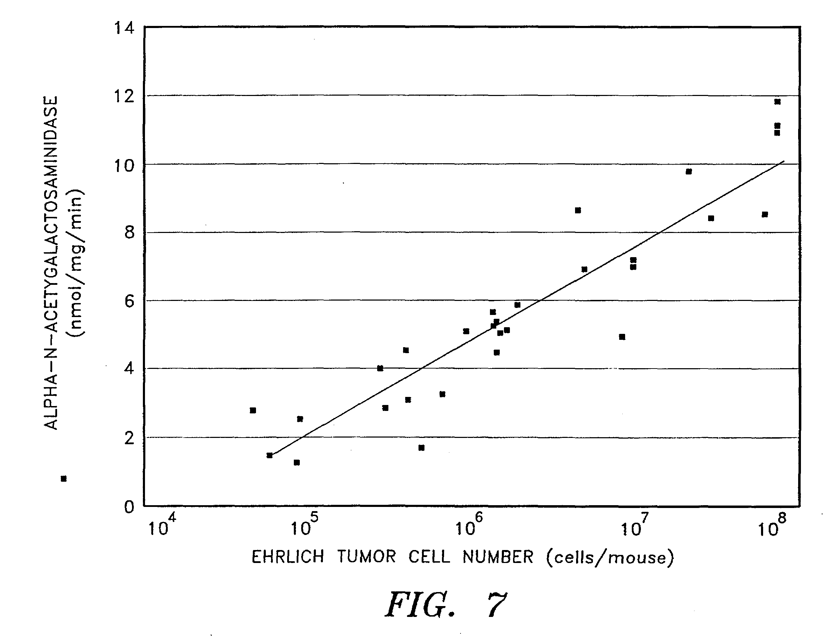

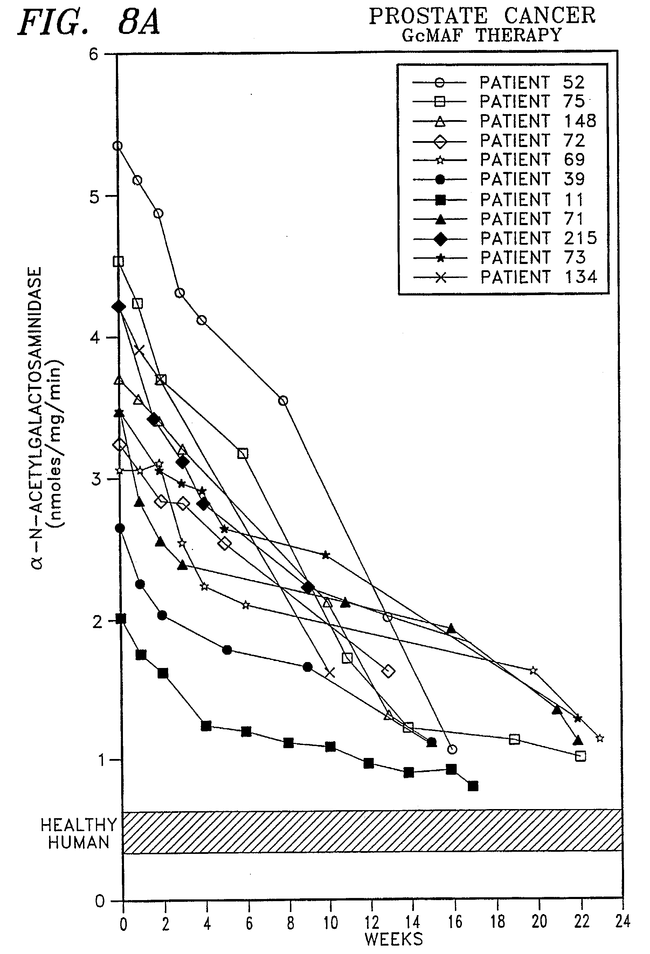

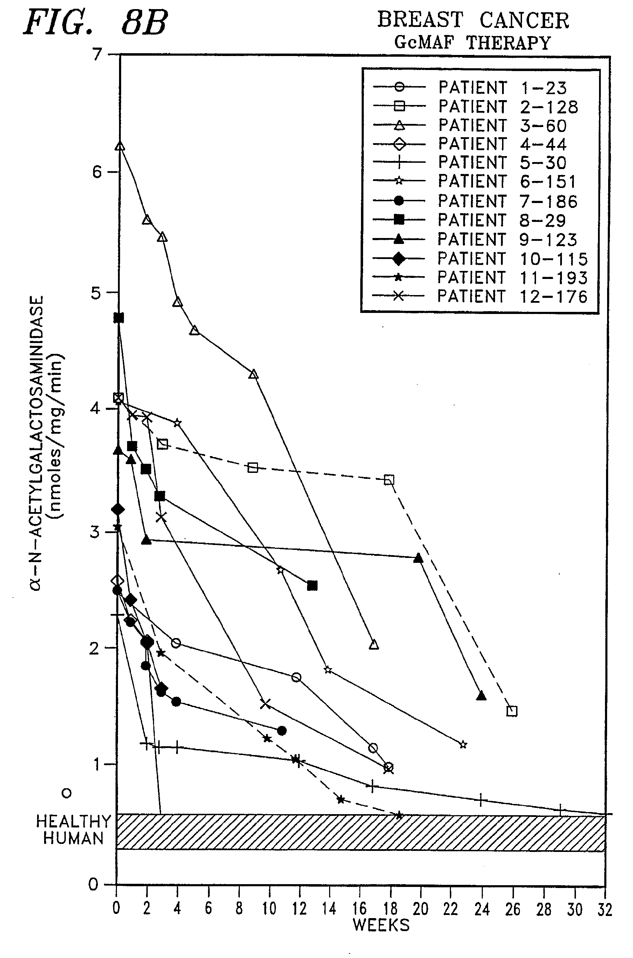

The dishes were incubated at room temperature for 4 hours with slow rocking. After transfection, the 1 ml of medium was added and incubated at 27°C in a humidified environment for 48 hours. The resultant transfection lysate was plaque assayed. Purification of recombinant virus, isolation of the cloned domain peptide (Cd₂) and enzymatic generation of the cloned macrophage activating factor designated CdMAF₂ were described as in the Cloning Procedure I. This CdMAF is composed of 94 amino acid residues as shown in Fig. 6, including 9 amino acids from the fusion vector and is referred to herein as CdMAF₂. Although CdMAF₂ has five amino acids more than the CdMAF₁ peptide derived from the non-fusion vector, they exhibited the same biological activities.

### DESCRIPTION OF THE ASSAY METHODS FOR α-N-ACETYLGALACTOSAMINIDASE ACTIVITY IN BLOOD STREAM OF CANCER AND HIV-INFECTED PATIENTS

### 1. Assay protocol for detection of α-N-acetylgalactosaminidase in blood stream of cancer and HIV-infected/AIDS patients (Schematic illustration)

Detection procedure for deglycosylating enzyme of serum Gc protein, α-N-acetylgalactosaminidase, in the bloodstream of cancer patients and HIV-infected/AIDS patients.

### Step. I. Stepwise 30 and 70% ammonium sulfate precipitation of plasma or serum:

**Plasma/sera (1 ml) + 30% and then 70% saturated ammonium sulfate**
70% precipitate --> dissolved in 50 mM citrate phosphate buffer (pH 6.0) --> dialyzed against the same buffer at 4°C for overnight.

### Step. II. Enzyme assay of α-N-acetylgalactosaminidase

Reaction mixture: 100 µl of the dialyzed sample + 1.0 ml of 50 mM citrate phosphate buffer (pH 6.0) containing 5 µmoles of
p-nitrophenyl N-acetyl-α-D-galactosaminide as substrate.
Incubation time: 60 min, terminated by adding 200 µl of 0.5 M Na₂CO₃.
Activity measurement: absorbance of amount of released
p-nitrophenol at 420 nm and expressed as nmole/mg/min.

### 2. Enzymatic assay procedure for α-N-acetylgalactosaminidase.

Plasma/serum (1 ml) of a healthy human and patients is precipitated with 70% saturated ammonium sulfate. The ammonium sulfate precipitate is dissolved in 50 mM citrate phosphate buffer (pH 6.0) and dialyzed against the same buffer at 4°C. The volume of the dialysate is made up to 1 ml. Ammonium sulfate precipitation separates the enzyme from inhibitors. The enzyme activity is determined at 37°C in a reaction mixture of 500 µl containing 50 mM citrate phosphate buffer (pH 6.0) and 5 µmoles of p-nitrophenyl N-acetyl-α-D-galactosaminide as a substrate. The reaction is initiated by addition of 250 µl of the dialyzed samples and stopped after 60 min by adding 250 µl of 0.5 M Na₂CO₃ solution. The reaction mixture is centrifuged and amount of released p-nitrophenol will be determined by the absorbance of the supernatant at 420 nm and expressed as nmole/mg/min.

### 3. Eanzyme-linked immunosorbent assay (ELISA) for detection of α-N-acetylgactosaminidase as an antigen.

In the immunoassay procedure for detection of serum or plasma α-N-acetylgalactosaminidase (Nag) as an antigen (NagAg), an antibody-sandwich ELISA kit was prepared.
**1) Preparation of antibody.** Polyclonal antibodies (goat and rabbit) and monoclonal antibodies against α-N-acetylgalactosaminidase (NagAg) were prepared. Immunoglobulin fraction was purified from antisera or monoclonal ascites fluid using ammonium sulfate (50% saturated) fractionation and DE52 ionic exchange or protein A columns.
**2) Conjugation of alkaline phosphatase to antibodies.** Dialyze 5 mg/ml antibody solution in 0.1 M phosphate buffer at pH 6.8 (PBS) overnight at 4°C. Add 100 µl of dialyzed antibody solution (3 mg/ml) to 90 µl of 10 mg/ml alkaline phosphatase (immunoassay grade; Boehringer Mannheim, Indianapolis, IN) in a 1.5-ml microcentrifuge tube. Add 5 µl 25% glutaraldehyde and mix gently. Let stand at room temperature. Remove 25-µl samples at time 0, 5, 10, 15, 30, 60 and 120 min and place a separate 1.5-ml microcentrifuge tubes. Add 125 µl PBS to each sample, then add 1.1 ml Tris/ovoalbumin solution. Dialyze samples against PBS overnight at 4°C. Test each sample for alkaline phosphatase activity using a direct ELISA to determine which conjugation time yields the most active enzyme conjugation. A 30 min conjugation time yielded the best enzyme activity. This optimal conjugation time was used for preparation of a larger amount of alkaline phosphatase-antibody conjugates.
**3) Preparation of antibody coated microtiter plates.** Using multichannel pipets and tips, dispense 50 µl of immunoglobulin solution 2 to 10 µg/ml in PBSN (PBS containing 0.05% sodium azide) into each well of a microtiter plate (microwell). Wrap the plates in plastic wrap to seal and incubate 2 hrs at 37°C or overnight at room temperature. Rinse the coated plate by flooding with distilled water more than three times. Fill each well with blocking buffer (PBS containing 0.05% tween 20, 1 mM EDTA, 0.25% BSA and 0.05% NaN₃) dispensed as a stream from a bottle and incubate 30 min at room temperature. Rinse the plate three times with distilled water and remove the residual liquid by gently flicking it face down onto paper towels.
**4) Antigen (NagAg) preparation.**
   **(a) Standard antigen for standard curve.** Prepare a standard antigen-dilution series by successive 1:3 dilutions of antigen stock in blooking buffer. The range of dilution spans from 0.1 to 1000 ng/ml.
   **(b) Test antigen.** Dilute the scrum/plasma of cancer or HIV-infected patients 10- to 100-fold because they contain 20 to 1000 ng of apoprotein/ml of patient serum/plasma.
**5) Assay:**
   **Step I.** Add 50-µl aliquots of the antigen test solutions and the standard antigen dilutions to the antibody coated wells and incubate 2 hrs at room temperature. Rinse the plate three times in distilled water. Fill each well with blocking buffer and incubate 10 min at room temperature. Rinse three times with water and remove the residual liquid.
   **Step II.** Add 50 µl specific antibody-alkaline phosphatase conjugate (typically 25 to 400 ng/ml) and incubate 2 hr at room temperature. Wash the plate as in Step I.
   **Step III.** Add 75 µl of p-nitrophenyl phosphate (NPP) substrate solution to each well and incubate 1 hr at room temperature. Enzyme activity causes the solution to change color. Color intensity relates to the presence or absence of NagAg. Read the plate on a microtiter plate reader with a 405-nm filter.
**6) Data analysis.** Prepare a standard curve constructed from the data produced by serial dilutions of the standard antigen. Plot the antigen concentration on the x axis which is a log scale, and absorbance on the y axis which is a linear scale. Interpolate the concentration of antigen (NagAg) in the test solution. Calculate the α-N-acetylgalactosaminidase activity. Since antibodies to cancer and HIV enzymes are specific to the respective enzymes (NagAg), this antibody sandwich ELISA distinguishes the individual enzyme as well as the antigen (α-galactosidase) in the control. Serum/plasma α-N-acetylgalactosaminidase activity was also determined by interpolating the NagAg concentration in another standard curve prepared for the α-N-acetylgalactosaminidase activity vs. NagAG concentration. This ELISA assay for the enzyme activity is not only accurate but also economical.

### SUPPORTING OBSERVATIONS

### A. Effects of Cloned Macrophage Activating Factors, GcMAFc and CDMAF on Cultured Phagocytes (Macrophages and Osteoclasts).

The three hour treatment of human macrophages and osteoclasts with picogram quantities (pg) of the cloned macrophage activating factors, GcMAFc and CdMAF, resulted in a greatly enhanced superoxide generating capacity of these phagocytes as shown in Table 1. The levels of the phagocyte activation are similar to those of macrophage activation by GcMAF (Yamamoto et al., AIDS Res. Human Ret. 11:1373, 1995).

**Table 1.**

| Activation of phagocytes by in vitro treatment with GcMAF and its cloned derivatives. | | | | |
|---|---|---|---|---|
| | Conc. pg/ml | nmole of superoxide produced/min/10⁶ phagocytes | | |
| | | Human macrophages* | Mouse peritoneal macrophages | Human osteoclasts |
| GcMAFc | 0 | 0.07 | 0.06 | 0.03 |
| | 10 | 3.20 | 3.46 | 2.56 |
| | 100 | 5.18 | 5.08 | 4.22 |
| | | | | |
| CdMAF | 0 | 0.01 | 0.02 | 0.08 |
| | 10 | 2.96 | 2.87 | 2.43 |
| | 100 | 4.26 | 4.53 | 4.09 |

| | | | | |
|---|---|---|---|---|
| *Peripheral blood monocytes/macrophages of cancer patients. Similar results were also observed when those of HIV-infected patients were used. | | | | |

### B. Activation of Mouse Peritoneal Macrophages by Administration of Cloned Macrophage Activating Factors, GcMAFc and CdMAF.

One day post-administration of a picogram quantity (10 and 100 pg/mouse) of GcMAFc or CdMAF to BALB/c mice, peritoncal macrophages were isolated and assayed for superoxide generating capacity. As shown in Table 2, the macrophages were efficiently activated. These results are similar to those of macrophage activation with GcMAF (Naraparaju and Yamamoto, Immunol. Lett. 43:143, 1994).

**Table 2.**

| Activation of mouse peritoneal macrophages by administration of cloned GcMAF derivatives. | | |
|---|---|---|
| | Dosage pg/mouse | nmole of superoxide produced/min/10⁶ phagocytes |
| | | Mouse peritoneal macrophages |
| GcMAFc | 0 | 0.05 |
| | 10 | 3.18 |
| | 100 | 5.23 |
| | | |
| CdMAF | 0 | 0.03 |
| | 10 | 2.54 |
| | 100 | 4.23 |

### C. Therapeutic Effects of GcMAF, GcMAFc or CdMAF on Tumor Bearing Mice and Osteopetrotic Mice.

### 1) Therapeutic effects of GcMAF, GcMAFc or CdMAF on Ehrlich ascites tumor bearing mice.

When BALB/c mice were administered with GcMAF, GcMAFc or CdMAF (100 pg/mouse) and received 10⁵ Ehrlich ascites tumor cells/mouse, they survived for at least 5 weeks. All the control mice received only the ascites tumor and died in approximately 2 weeks. When mice were administered with an additional GcMAF, GcMAFc or CdMAF (100 pg/mouse) 4 days post-transplantation, the tumor cells were completely eliminated (Table 3).

**Table 3.**

| Therapeutic effects of GcMAF and cloned derivatives on mice bearing Ehrlich ascites tumor. | | | |
|---|---|---|---|
| Group | No. of mice | Post-transplantation treatment^{a} | No. of mice survived/period |
| Group 1. | | | |
| | 6 mice | untreated control | 6 mice/13 ± 3 days |
| | 10 mice | day 0 100 pg GcMAF/mouse | 10 mice/36 ± 7 days |
| | | | |

| Group 2. | | | |
|---|---|---|---|
| | 6 mice | untreated control | 6 mice/14 ± 4 days |
| | 10 mice | day 0 100 pg GeMAFc/mouse | 10 mice/35 ± 6 days |

| Group 3. | | | |
|---|---|---|---|
| | 6 mice | untreated control | 6 mice/14 ± 5 days |
| | 10 mice | day 0 100 pg CdMAF/mouse | 10 mice/34 ± 3 days |
| | | | |

| Group 4. | | | |
|---|---|---|---|
| | 8 mice | untreated control | 8 mice/15 ± 5 days |
| | 12 mice | day 0 100 pg GcMAF/mouse | |
| | day 4 | 100 pg GcMAF/mouse | 12 mice/ >65 days |
| | | | |

| Group 5. | | | |
|---|---|---|---|
| | 8 mice | untreated control | 8 mice/14 ± 5 days |
| | 12 mice | day 0 100 pg GcMAFc/mouse | |
| | | day 4 100 pg GcMAFc/mouse | 12 mice/ >65 days |
| | | | |

| Group 6. | | | |
|---|---|---|---|
| | 8 mice | untreated control | 8 mice/14 ± 5 days |
| | 12 mice | day 0 100 pg CdMAF/mouse | |
| | | day 4 100 pg CdMAF/mouse | 12 mice/ >65 days |

| | | | |
|---|---|---|---|
| ^{a}GcMAF, mice in all GcMAFc and CdMAF were administered intramuscularly (systemically) and groups receive 10⁵ tumor cells/mouse. | | | |

### 2) Therapeutic effects of GcMAF and cloned GcMAF derivatives (GcMAFc and CdMAF) on osteopetrotic mice.

The administration of GcMAFc or CdMAF to new born litters of osteopetrotic op/op mouse was performed by the weekly injection of 100 picograms for four weeks beginning from a day after birth. Mice were sacrificed at 28 days. The tibiae were removed from the treated and untreated control mice, longitudinally bisected, and examined under a dissecting microscope to measure the size of the bone marrow cavity. The cavity size was expressed as a percentage of the distance between the epiphyseal plates of the tibia. The untreated mouse group formed bone marrow with 30% of the total length of tibia. The treated mouse group experienced a 20% increased bone marrow formation over that of the untreated mouse group. This increased bone marrow cavity formation is an indication of osteoclast activation and increased os-teoclastic bone resorption.

### D. The Origin or Immunosuppression in Cancer and HIV-infected Patients.

The source of the plasma α-N-acetylgalactosaminidase in cancer patients appeared to be cancerous cells. High α-N-acetylgalactosaminidase activities were detected in tumor tissue homogenates of various organs, including eleven different tumor tissues including 4 lung, 3 breast, 3 colon and 1 cervix tumors, though the α-N-acetylgalactosaminidase activity varied from 15.9 to 50.8 nmoles/mg/min. Surgical removal of malignant lesions in human cancer results in the subtle decrease of plasma α-N-acetylgalactosaminidase activity with th;e concomitant increase in the precursor activity, particularly if malignant cells are localized.

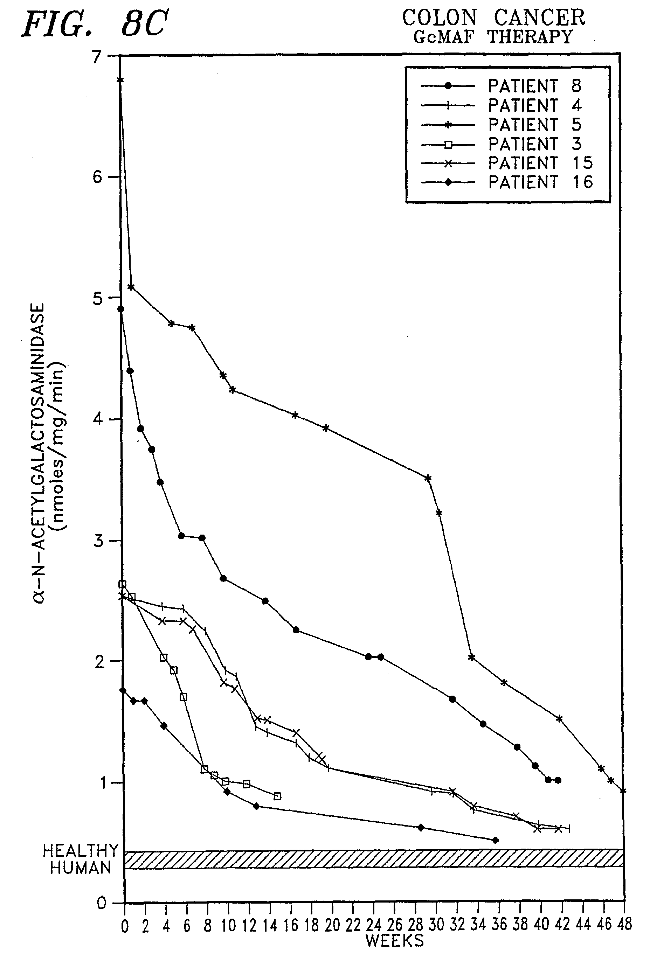

In a preclinical mouse tumor model, BALB/c mice were transplanted with 5 X 10⁵ Ehrlich ascites tumor cells/mice into peritoneal cavity and analyzed for serum α-N-acetylgalactosaminidase activity. When serum enzyme levels were measured as transplanted Ehrlich ascites tumor grew in the mouse peritoneal cavity, the enzyme activity was directly proportional to the tumor burden (the total cell counts in peritoneal cavity) as shown in Fig. 7. This was also confirmed with nude mice transplanted with KB cells (human oral squamous cell carcinoma cell line). Serum α-N-acetylgalactosaminidase activity increased as tumor size (measured by weight) of the solid tumor increased. Thus, I have been using plasma α-N-acetylgalactosaminidase activity as a prognostic index to monitor the progress of therapy.

Radiation therapy of human cancer decreased plasma α-N-acetylgalactosaminidase activity with a concomitant increase of precursor activity. This implies that radiation therapy decreases the number of cancerous cells capable of secreting α-N-acetylgalactosaminidase. These results also confirmed that plasma α-N-acetylgalactosaminidase activity has an inverse correlation with the MAF precursor activity of Gc protein. Even after surgical removal of tumor lesions in cancer patients, most post-operative patients carried significant amounts of α-N-acetylgalactosaminidase activity in their blood stream. The remnant cancerous lesions in these post-operative patients cannot be detectable by any other procedures, e.g., X-ray, scintigraphy, etc. I have been using this most sensitive enzyme assay as a prognostic index during the course of GcMAF therapy.

HIV-infected cells appeared to secrete α-N-acetylgalactosaminidase into the blood stream. When peripheral blood mononuclear cells (PBMC) of HIV-infected patients were cultured and treated with mitomycin as a provirus inducing agent (Sato et al., Arch. Virol. 54:333, 1977), α-N-acetylgalactosaminidase was secreted into the culture media. These results led me to suggest that α-N-acetylgalactosamirudase is a virus coded product. In fact, HIV-envelope protein gp120 appears to carry the α-N-acetylgalactosaminidase activity.

### E. Therapeutic Effects of GcMAF, GcMAFc and CdMAF on Human Cancer and Virus Infected Patients.

### 1. Cancer patients: Therapeutic effect of GcMAF on prostate, breast and colon cancer and adult leukemia patients.

The administration of GcMAF (100 and 500 ng/human) to healthy volunteers resulted in the greatly enhanced activation of macrophages as measured by the 7-fold enhanced phagocytic capacity and the 15-fold superoxide generating capacity of macrophages. The administration of GcMAF showed no signs of any side effects to the recipients. Administration of various doses (100 pg to 10 ng/mouse) to a number of mice produced neither ill effects nor histological changes in various organs including liver, lung, kidney, spleen, brain, etc. When patients with various types of cancer were treated with GcMAF (100 ng/week), remarkable curative effects on various types of cancer were observed. The therapeutic efficacy of GcMAF on patients bearing various types of cancers was assessed by tumor specific serum α-N-acetylgalactosaminidase activity because the serum enzyme level is proportional to the total amount of cancerous cells (tumor burden). Curative effects of GcMAF on prostate, breast and colon cancer are illustrated in Figs. 8a to 8c. After 25 weekly administrations of 100 ng GcMAF the majority (>90%) of prostate and breast cancer patients exhibited insignificantly low levels of the serum enzyme. A similar result was also observed after 35 GcMAF administrations to colon cancer patients. Similar curative effects of GcMAF on lung, liver, stomach, brain, bladder, kidney, uterus, ovarian, larynx, esophagus, oral and skin cancers were observed. Thus, GcMAF appeared to be effective on a variety of cancers indiscriminately. However, GcMAF showed no evidence of side effects in patients after more than 6 months of therapy. This was also confirmed by blood cell count profile, liver and kidney functions, etc.

When GcMAFc (100 ng/week) and CdMAF (100 ng/week) were administered to two prostate cancer patients each, curative effects similar to those of GcMAF were observed.

### 2. Virus infected patients

Treatment of peripheral blood macrophages of HIV-infected/AIDS patients with 100 pg GcMAF/ml resulted in a greatly enhanced macrophage activation (Yamamoto et al., AIDS Res. Human Ret. 11:1373, 1995). HIV-infected patients carry anti-HIV antibodies. HIV-infected cells express the viral antigens on the cell surface. Thus, macrophages have a potential to eliminate the infected cells via Fc-receptor mediated cell-killing/ingestion when activated.

Similarly, treatment of peripheral blood macrophages of patients chronically infected with Epstein-Barr virus (EBV) and with herpes zoster with 100 ng GcMAF/ml resulted in a greatly enhanced macrophage activation. Like HIV, EBV infects lymphocytes (B cells). Since these enveloped viruses code for α-N-acetylgalactosaminidase and infected cells secrete it into blood stream, this enzyme activity in patient sera can be used as a prognostic index during therapy. After approximately 25 administrations of GcMAF (100 ng/week) to patients chronically infected with EBV and with herpes zoster, the enzyme activity decreased to that of healthy control levels. When GcMAFc (100 ng/week) and CdMAF (100 ng/weck) were administered to EBV-infected patients, curative effects similar to those of GcMAF were observed.

### F. Adjuvant Activities of GcMAF, GcMAFc and CdMAF for Immunization and Vaccinations: Rapid increase of the number of antibody secreting cells (PFC) in mice after administration of GcMAF and sheep erythrocytes.

BALB/c mice were inoculated with SRBC 6 hours after the intraperitoneal administration of 50 pg GcMAF/mouse. At various intervals (1-5 days) after immunization, IgM-antibody secreting cells in the spleen were determined using the Jerne plaque assay (Jerne et al., Cell-bound antibodies, Wistar Institute Press, 1963). One day post-administration of GcMAF and SRBC produced 1.35 x 10⁴ PFC/spleen. Two days after administration of GcMAF and SRBC, the number of antibody secreting cells had increased to 8.2 x 10⁴ PFC/spleen. By the 4th day the number of antibody secreting cells reached the maximal level (about 23.6 x 10⁴ PFC/spleen), as shown in Table 4. In contrast, mice that received an injection of SRBC alone produced about 3.8 x 10⁴ PFC/spleen, 4 days after SRBC-injection.

To ascertain the dose response, mice were injected with SRBC 6 hours after administration of various doses of GcMAF ranging from 1 to 50 pg/mouse. On the 4th day post-administration of GcMAF and SRBC, the number of antibody secreting cells per spleen was determined by the Jerne plaque assay. On the 4th day post-administration there was a commensurate increase in the number of plaque forming cells as the concentration of GcMAF was increased above 1 pg per mouse. At a GcMAF dose of 5, 10 and 50 pg/mouse, I detected 12.6 X 10⁴, 20.2 x 10⁴ and 24.3 X 10⁴ PFC/spleen, respectively. Therefore, GcMAF is a potent adjuvant for immunization.

**Table 4.**

| Time course studies on development of cells secreting antibody against sheep erythrocytes (SRBC) in BALB/c mice after administration of GcMAF and SRBC^{a}. | | |
|---|---|---|
| After SRBC immunization (days) | Antibody secreting cells/spleen (X10⁴) | |
| | SRBC only | GcMAF + SRBC |
| 1 | 0.01 ± 0.002 | 1.35 ± 0.21 |
| 2 | 0.08 ± 0.02 | 8.28 ± 1.26 |
| 3 | 1.18 ± 0.42 | 14.42 ± 2.32 |
| 4 | 3.86 ± 0.95 | 23.68 ± 6.05 |
| 5 | 2.15 ± 0.63 | 18.63 ± 3.43 |

| | | |
|---|---|---|
| ^{a}Mice were inoculated with SRBC (10⁸ cells) 6 hr after administration of GcMAF (50 pg/mouse). The number of plaques (IgM secreting cells) was quantified microscopically on various days post-SRBC injection. The number of plaque-forming cells (PFC) per spleen is expressed as the mean value of triplicate assays ± SEM. | | |

Without further elaboration the foregoing will so fully illustrate my invention that others may, by applying current or future knowledge, adapt the same for use under various conditions of service.

### References Cited

The following references are cited and their entire text is incorporated fully herein as are all references set forth above in the specification.

### U.S. PATENT DOCUMENTS

U.S. Patent Nos. 5,177,001, 5,177,002 and 5,326,749 (Yamamoto).

### OTHER PUBLICATIONS

1. Jerne, N. K., Nordin, A. A. and Henry, C, The agar plaque technique for recognizing antibody producing cells. In Amos and Koprowski (eds). Cell-bound Antibody. Wistar Institute Press, Philadelphia, PA (1963).
2. Sato, M., Tanaka, H., Yamada, T. and Yamamoto, N., Persistent infection of BHK/WI-2 cells with rubella virus and characterization of rubella variants. Arch. Virology 54:333-343 (1977).
3. Reeves, J. D., August, C. S., Humbert, J. R., Weston, W. L, Host defense in infantile osteoporosis. Pediatrics. 64:202- (1979).
4. Marks, S. C., Jr., Congenital osteopetrotic mutations as probes of the origin, structure and function of osteoclasts. Clin. Orthop. 189:239- (1984).
5. Link, R. P., Perlman, K. L, Pierce, E. A., Schnoes, H. K., DeLuca, H. F. Purification of human serum vitamin D-binding protein by 25-hydroxyvitamin D₃,-Sepharose chromatography. Anal. Biochem. 157:262-269 (1986).
6. Cooke, N. E. and Haddad, J. G., Vitamin D binding protein (Gc-globulin). Endocrine Rev. 10:294-307 (1989).
7. Marks, S. C., Jr., Osteoclast biology: Lessons from mammalian mutations. Am. J. Med. Genet. 34:43-54 (1989).
8. Ngwenya, B.Z. and Yamamoto, N., Contribution of lysaphosphatidylcholine treated nonadherent cells to mechanism of macrophage stimulation. Proc. Soc. Exp. Biol. Med. 193:118-124 (1990).
9. Ausubel, F. A., Brent, R., Kingston, R. E., Moore, D. D., Seidman, J. G., Smith, J. A. and Struhl, K. (eds.), Expression of proteins in insect cells using baculoviral vectors. Current Protocols in Molecular Biology. Sections 16.8.1-16.11.7. Greene Publishing and Wiley-Interscience, New York (1990).
10. Yamamoto, N. and Homma, S., Vitamin D₃ binding protein (group-specific component, Gc) is a precursor for the macrophage activating signal from lysophosphatidylcholine-treated lymphocytes. Proc. Natl. Acad. Sci. USA. 88:8539-8543 (1991).
11. Cooke, N. E. and David, E. V., Serum vitamin D-binding protein is a third member of the albumin and alpha-fetoprotein gene family. J. Clin. Invest. 76:2420-2424 (1985).
12. Haddad, J. G., Hu, Y. Z., Kowalski, M. A., Laramore, G, Ray, K, Robzyk, P. and Cooke, N. E., Identification of the sterol- and actin-binding domains of plasma vitamin D binding protein (Gc-globulin). Biochemistry 31:7174-7181 (1992).
13. Yamamoto, N. and Kumashiro, R., Conversion of vitamin D₃ binding protein (Group-specific component) to a macrophage activating factor by stepwise action of β-galactosidase of B cells and sialidase of T cells. J. Immunol. 151:27-94-2902 (1993).
14. Yamamoto, N., Kumashiro, R., Yamamoto, M., Willett, N. P. and Lindsay, D.D., Regulation of inflammation-primed activation of macrophages by two serum factors, vitamin D₃-binding protein and albumin. Inf. Imm. 61:5388-5391 (1993).
15. Yamamoto, N., Lindsay, D. D., Naraparaju, V. R., Irelalnd, R. A. and Popoff, S.M., A defect in the inflammation-primed macrophage activation cascade in osteopetrotic (op) rats. J. Immunol. 152:5100-5107 (1994).
16. Yamamoto, N., Willett, N.P. and Lindsay, D.D., Participation of serum proteins in the inflammation-primed activation of macrophages. Inflammation. 18:311-322 (1994).
17. Naraparaju, V. R. and Yamamoto, N., Roles of β-galactosidase of B lymphocytes and sialidase of T lymphocytes in inflammation-primed activation of macrophages. Immunol. Lett. 43:143-148 (1994).
18. Yamamoto, N., Naraparaju, V.R. and Srinivasula, S.M., Structural modification of serum vitamin D₃-binding protein and immunosuppression in HIV-infected patients. AIDS Res. Human Ret. 11:1373-1378 (1995).

## Claims

1. A process for producing a cloned macrophage activating factor (GcMAFc) comprising:
a) Cloning a Gc1 isoform into a baculovirus vector;
b) Expressing the cloned Gc1 isoform thereby producing the cloned protein (Gc1), wherein the Gc1 protein comprises approximately 458 amino acids with a molecular weight of approximately 52000 Da and three distinct domains,
c) contacting cloned Gc protein *in vitro* with immobilized β-galactosidase and sialidase; and
d) obtaining the cloned macrophage activating factor (GcMAFc).
